(19) Europäisches Patentamt
European Patent Office
Office européen des brevets

(11) Veröffentlichungsnummer: **0 116 360**
**B1**

# (12) EUROPÄISCHE PATENTSCHRIFT

(45) Veröffentlichungstag der Patentschrift: **01.07.87**

(51) Int. Cl.⁴: **C 07 D 209/42**, A 61 K 31/405

(21) Anmeldenummer: **84101179.4**

(22) Anmeldetag: **06.02.84**

## (54) 1-Phenyl-2-aminocarbonylindol-Verbindungen sowie Verfahren zu ihrer Herstellung und diese Verbindungen enthaltende Arzneimittel.

(30) Priorität: **07.02.83 DE 3304019**

(43) Veröffentlichungstag der Anmeldung: **22.08.84 Patentblatt 84/34**

(45) Bekanntmachung des Hinweises auf die Patenterteilung: **01.07.87 Patentblatt 87/27**

(84) Benannte Vertragsstaaten: **BE CH DE FR GB IT LI NL**

(56) Entgegenhaltungen:
**EP-A-0 071 935**
**DE-A-3 304 019**
**FR-M-1 527**
**FR-M-5 723**

(73) Patentinhaber: **Kali- Chemie Pharma GmbH, Hans- Böckler- Allee 20 Postfach 220, D-3000 Hannover 1 (DE)**

(72) Erfinder: **Ohlendorf, Heinrich- Wilhelm, Dr. Dipl.- Chem., Schuhmachersweg 18, D-3008 Garbsen 2 (DE)**
Erfinder: **Kaupmann, Wilhelm, Dr. Dipl.- Chem., St. Ingbert- Weg 9, D-3000 Hannover 71 (DE)**
Erfinder: **Kühl, Ulrich G., Dr.med.vet., Franzburger Strasse 10, D-3007 Gehrden 7 (DE)**
Erfinder: **Buschmann, Gerd, Dr. med.vet., Matthäikirchstrasse 27, D-3000 Hannover (DE)**
Erfinder: **Magda, Stephen John, Dr. Dipl.- Chem., Gellertstrasse 24, D-3000 Hannover 1 (DE)**

(74) Vertreter: **Lauer, Dieter, Dr., c/o Kali- Chemie AG Postfach 220 Hans- Böckler- Allee 20, D-3000 Hannover 1 (DE)**

**EP 0 116 360 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

1-Phenyl-2-aminocarbonylindol-Verbindungen sowie Verfahren zu ihrer Herstellung und diese Verbindungen enthaltende Arzneimittel

Die vorliegende Erfindung betrifft neue 3-Acyloxy-1-phenyl-2-alkylaminocarbonylindol-Verbindungen und deren Salze sowie diese Verbindungen enthaltende pharmazeutische Zubereitungen.

Das belgische Patent Nr. 701023 und das franzosische Patent Nr. 1 503 908 beschreiben 2-Carboxamidoindol-Verbindungen, welche antiemetische und zentraldämpfende Wirkungen zeigen, und die französischen Patente Nr. 1527M und 1 313 759 beschreiben 2-Carboxamidoindol-Verbindungen, welche ebenfalls antiemetische und daneben lokalanästhesierende und antifibrillatorische Eigenschaften besitzen.

Die erst nach dem Prioritätsdatum der vorliegenden Anmeldung publizierte europäische Patentanmeldung 71 935 beschreibt antiarrhythmisch wirksame 3-Alkoxy-1-phenyl-2-alkylaminocarbonylindol-Verbindungen, von denen sich die vorliegenden neuen Verbindungen durch ihre andersartige Substitution in 3-Stellung unterscheiden.

Der Erfindung liegt die Aufgabe zugrunde, neue 1-Phenyl-2-aminocarbonylindol-Verbindungen mit wertvollen pharmakologischen Eigenschaften zu entwickeln.

Es wurde nun gefunden, daß die neuen in 3-Stellung durch Acyloxy substituierten 1-Phenyl-2-aminocarbonylindol-Verbindungen wertvolle pharmakologische Wirkungen, insbesondere antiarrhythmische Wirkungen, besitzen und ein vorteilhaftes Wirkungsprofil mit guter therapeutischer Breite und geringer Toxizität aufweisen. Aufgrund dieser Wirkungen sind die neuen Verbindungen als Arzneimittel, insbesondere zur Behandlung von Herz-Rhythmusstörungen geeignet.

Die vorliegende Erfindung betrifft daher neue 1-Phenyl-2-aminocarbonylindol-Verbindungen der allgemeinen Formel

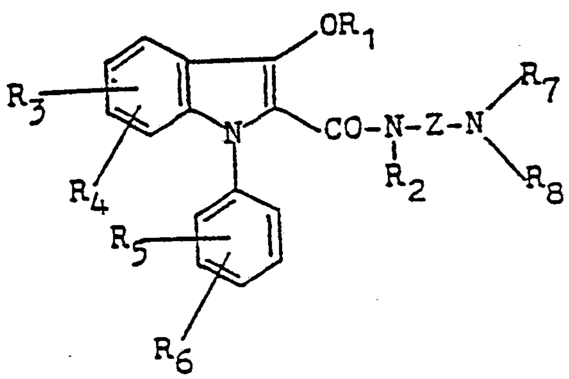

I

worin

$R_1$ eine Alkanoylgruppe mit 2 bis 12 Kohlenstoffatomen bedeutet welche gegebenenfalls substituiert sein kann durch einen Alkoxyrest mit 1 bis 4 Kohlenstoffatome oder eine Carboxyl- oder Alkoxycarbonylgruppe, wobei die Alkoxycarbonylgruppe einen Alkoxyrest mit 1 bis 4 Kohlenstoffatom besitzt, oder

$R_1$ eine Gruppe a

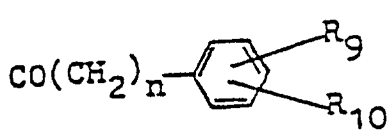

a

bedeutet, worin n 0, 1, 2 oder 3 ist und $R_9$ und $R_{10}$ unabhängig voneinander Wasserstoff, Halogen, Alkyl mit 1 bis 4 Kohlenstoffatomen oder Alkoxy mit 1 bis 4 Kohlenstoffatomen oder, falls sie an 2 benachbarte Kohlenstoffatome gebunden sind, auch gemeinsam Methylendioxy darstellen, oder, falls n 0 ist und $R_9$

Wasserstoff darstellt, $R_{10}$ auch Nitro oder Trifluormethyl bedeutet, oder

$R_2$ Wasserstoff oder Alkyl mit 1 bis 4 Kohlenstoffatomen bedeutet,

$R_3$ Wasserstoff, Halogen, Alkyl mit 1 bis 4 Kohlenstoffatomen oder Alkoxy mit 1 bis 4 Kohlenstoffatomen bedeutet,

$R_4$ Wasserstoff, Halogen, Alkyl mit 1 bis 4 Kohlenstoffatomen oder Alkoxy mit 1 bis 4 Kohlenstoffatomen bedeutet,

$R_5$ Wasserstoff, Alkyl mit 1 bis 4 Kohlenstoffatomen, Halogen oder Alkoxy mit 1 bis 4 Kohlenstoffatomen bedeutet,

$R_6$ Wasserstoff, Alkyl mit 1 bis 4 Kohlenstoffatomen, Alkoxy mit 1 bis 4 Kohlenstoffatomen, Halogen oder, falls $R_5$ Wasserstoff ist, auch Nitro oder Trifluormethyl bedeutet,

$R_7$ Wasserstoff oder Alkyl mit 1 bis 4 Kohlenstoffatomen bedeutet,

$R_8$ Wasserstoff oder Alkyl mit 1 bis 4 Kohlenstoffatomen bedeutet, oder

$R_7$ und $R_8$ zusammen mit dem Stickstoffatom, an das sie gebunden sind, eine heterocyclische Gruppe b bilden,

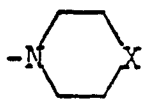

b

worin

X für eine Bindung, $-CH_2-$, $-C_2H_4-$, O oder S steht,

Z eine Alkylenkette mit 2 bis 5 Kohlenstoffatomen bedeutet, welche gegebenenfalls an einem nicht an Stickstoff gebundenen Kohlenstoff durch Hydroxy oder eine $R_1O$-Gruppe, worin $R_1$ die oben angegebene Bedeutung besitzt, substituiert ist,

sowie deren Säureadditionssalze.

Sofern in den Verbindungen der Formel I die Substituenten $R_3$ bis $R_6$ der Phenylringe eine Alkylgruppe enthalten, kann diese gerade oder verzweigt sein und 1 bis 4 Kohlenstoffatome enthalten. So kommen insbesondere Methyl, Äthyl, n-Propyl, Isopropyl, n-Butyl oder tert.-Butyl, bevorzugt Methyl oder Äthyl in Frage. Insbesondere bei Disubstitutionen an den Phenylringen stellen alkylhaltige Substituenten vorzugsweise Methyl oder Methoxy dar.

Als Halogensubstituenten in den Phenylringen kommen insbesondere Fluor, Chlor oder Brom, bevorzugt Chlor oder Brom, in Frage.

Die Substituenten $R_3$ und $R_4$ stellen vorzugsweise Wasserstoff oder Halogen dar. Die Substituenten $R_5$ und $R_6$ stellen vorzugsweise Wasserstoff, Halogen oder Alkyl dar.

Der Substituent $R_2$ steht insbesondere für Wasserstoff. Sofern $R_2$ Alkyl bedeutet, enthält dieses 1 bis 4, insbesondere 1 bis 2 Kohlenstoffatome.

Falls $R_7$ und/oder $R_8$ eine Alkylgruppe bedeuten, kann diese gerade oder verzweigt sein und 1 bis 4 Kohlenstoffatome enthalten. Als Alkylgruppen kommen insbesondere Methyl-, Äthyl-, Propyl- und Butyl-Gruppen in Frage. Insbesondere steht die $NR_7R_8$-Gruppe für eine vorzugsweise unverzweigte Dialkylaminogruppe, beispielsweise die Diäthylamino-Gruppe.

Z stellt eine Alkylenkette mit 2 bis 5 Kohlenstoffatomen dar, vorzugsweise eine gerade Alkylenkette mit 2 bis 4 Kohlenstoffatomen. Falls Z eine durch Hydroxy oder $R_1O$ substituierte Alkylenkette bedeutet, stellt diese vorzugsweise eine 2-Hydroxypropylen- oder 2-$R_1O$-Propylen-Kette dar.

Sofern die Acylgruppe $R_1$ niederes Alkyl enthaltende Substituenten trägt, besitzen diese insbesondere 1 bis 4 Kohlenstoffatome und stellen vorzugsweise Methyl oder Äthyl dar.

Die Acylgruppe $R_1$ stellt den Säurerest einer aliphatischen oder aromatischen Carbonsäure dar. Als aliphatische Carbonsäuren eignen sich niedere Monocarbonsäuren mit vorzugsweise 1 bis 4 Kohlenstoffatomen in der Alkylkette wie Essigsäure, Propionsäure oder Propan- und Butancarbonsäuren. Ferner eignen sich substituierte niedere Carbonsäuren mit vorzugsweise 1 bis 4 Kohlenstoffatomen in der Alkylkette, z. B. niedere Dicarbonsäuren wie Bernsteinsäure oder deren niedere Monoalkylester, sowie durch niederes Alkoxy, insbesondere Methoxy oder Äthoxy, oder Benzyloxy substituierte Monocarbonsäuren. Auch gegebenenfalls substituierte geradkettige oder verzweigte aliphatische Carbonsäuren mit bis zu 11 z. B. 5 bis 11 Kohlenstoffatomen in der Alkylkette sind geeignet wie beispielsweise Capronsäure, Caprylsäure oder Laurinsäure.

Als aromatische Säuren eignen sich gegebenenfalls substituierte Phenylalkylcarbonsäuren mit bis zu 3 Kohlenstoffatomen in der Alkylenkette und insbesondere gegebenenfalls substituierte Benzoesäuren.

Als Beispiele von geeigneten Acylresten $R_1$ seien genannt Acetyl, Propionyl, Isobutyryl, Valeroyl, Pivaloyl, Isovaleroyl, Dodecanoyl, Benzoyl, Chlorbenzoyl, Nitrobenzoyl, Methylbenzoyl, Methoxybenzoyl, Phenylacetyl, Hydroxycarbonyläthylcarbonyl, Methoxycarbonyläthylcarbonyl.

So gehören zu den erfindungsgemäßen Verbindungen unter anderen:.

2-[3-(N,N-Diäthylamino)-2-hydroxypropylaminocarbonyl]-3-pivaloyloxy-1-phenylindol,
2-[3-(N,N-Diäthylamino)-2-propionyloxypropylaminocarbonyl]-3-propionyloxy-1-phenylindol,
2-[3-(N,N-Diäthylamino)-2-hydroxypropylaminocarbonyl]-3-isobutyryloxy-1-phenylindol,
2-[3-(N,N-Diäthylamino)-2-hydroxypropylaminocarbonyl]-3-dodecanoyloxy-1-phenylindol,
2-[3-(N,N-Diäthylamino)-2-hydroxypropylaminocarbonyl]-3-benzoyloxy-1-phenylindol,
2-[3-(N,N-Diäthylamino)-2-hydroxypropylaminocarbonyl]-3-o-chlorbenzoyloxy-1-phenylindol,
2-[3-(N,N-Diäthylamino)-2-hydroxypropylaminocarbonyl]-3-m-chlorbenzoyloxy-1-phenylindol,
2-[3-(N,N-Diäthylamino)-2-hydroxypropvlaminocarbonyl]-3-p-chlorbenzovloxy-1-phenylindol,
2-[3-(N,N-Diäthylamino)-2-hydroxypropylaminocarbonyl]-3-p-nitrobenzoyloxy-1-phenylindol,
2-[3-(N,N-Diäthylamino)-2-hydroxypropylaminocarbonyl]-3-p-methylbenzoyloxy-1-phenylindol,
2-[3-(N,N-Diäthylamino)-2-hydroxypropylaminocarbonyl]-3-p-methoxybenzoyloxy-1-phenylindol,
2-[3-(N,N-Diäthylamino)-2-hydroxypropylaminocarbonyl]-3-m,p-dimethoxybenzoyloxy-1-phenylindol,
2-[3-(N,N-Diäthylamino)-2-hydroxypropylaminocarbonyl]-3-phenylacetoxy-1-phenylindol,
2-[3-(N,N-Diäthylamino)-2-hydroxypropylaminocarbonyl]-3-methoxycarbonylmethylcarbonyloxy-1-phenylindol,
2-[3-(N,N-Diäthylamino)-2-hydroxypropylaminocarbonyl]-3-hydroxycarbonyläthylcarbonyloxy-1-phenylindol,
2-[3-(N,N-Diäthylamino)-2-hydroxypropylaminocarbonyl]-3-methoxycarbonyläthylcarbonyloxy-1-phenylindol,
2-[3-(N,N-Diäthylamino)-2-hydroxypropylaminocarbonyl]-3-benzoyloxy-1-(4-chlorphenyl)-indol,
2-[3-(N,N-Diäthylamino)-2-hydroxypropylaminocarbonyl]-3-methoxyacetoxy-1-phenylindol,
2-[3-(N,N-Diäthylamino)-2-benzoyloxypropylaminocarbonyl]-3-benzoyloxy-1-(4-chlorphenyl)-indol,
2-[3-(N,N-Diäthylamino)-2-acetoxypropylaminocarbonyl]-3-acetoxy-1-phenylindol,
2-[3-(N,N-Diäthylamino)-2-benzoyloxypropylaminocarbonyl]-3-benzoyloxy-1-phenylindol,
2-[3-(N,N-Diäthylamino)-2-hydroxypropylaminocarbonyl]-3-pivaloyloxy-1-phenyl-5-bromindol,
2-[3-(N,N-Diäthylamino)-2-hydroxypropylaminocarbonyl]-3-pivaloyloxy-1-phenyl-4-chlorindol,
2-[3-(N,N-Diäthylamino)-2-hydroxypropylaminocarbonyl]-3-pivaloyloxy-1-(4-methoxyphenyl)-indol,
2-[3-(N,N-Diäthylamino)-2-hydroxypropylaminocarbonyl]-3-pivalovloxy-1-(4-fluorphenyl)-indol,
2-[3-(N,N-Diäthylamino)-2-hydroxypropylaminocarbonyl]-3-pivaloyloxy-1-(4-methoxyphenyl)-indol,
2-[3-(N,N-Diäthylamino)-2-propionyloxypropylaminocarbo-nyl]-3-propionyloxy-1-phenyl-5-bromindol,
2-[3-(N,N-Diäthylamino)-2-hydroxypropylaminocarbonyl]-3-benzoyloxy-1-phenyl-4-chlorindol,
2-[3-(N,N-Diäthylamino)-2-hydroxypropylaminocarbonyl]-3-benzoyloxy-1-(4-fluorphenyl)-indol,
2-[3-(N,N-Diäthylamino)-2-hydroxypropylaminocarbonyl]-3-pivaloyloxy-1-(4-chlorphenyl)-indol,
2-[3-(N,N-Diäthylamino)-2-hydroxypropylaminocarbonyl]-3-benzoyloxy-1-(4-methoxyphenyl)-indol,
2-(3-Pyrrolidino-2-hydroxypropylaminocarbonyl)-3-pivaloyloxy-1-phenylindol,
2-(3-Pyrrolidino-2-benzoyloxypropylaminocarbonyl)-3-benzoyloxy-1-phenylindol,
2-(3-Morpholino-2-hydroxypropylaminocarbonyl)-3-pivaloyloxy-1-phenylindol,
2-(3-Morpholino-2-hydroxypropylaminocarbonyl)-3-benzoyloxy-1-phenylindol,
2-[2-(N,N-Dimethylamino)-äthylaminocarbonyl]-3-acetoxy-1-phenylindol,
2-[3-(N,N-Diäthylamino)-propylaminocarbonyl]-3-acetoxy-1-phenylindol,
2-[4-(N,N-Diäthylamino)-butylaminocarbonyl]-3-acetoxy-1-phenylindol,
2-[2-(N,N-Dimethylamino)-äthylaminocarbonyl]-3-propionyloxy-1-phenylindol,
2-[3-(N,N-Diäthylamino)-propylaminocarbonyl]-3-propionyloxy-1-phenylindol,
2-[4-(N,N-Diäthylamino)-butylaminocarbonyl]-3-propionyloxy-1-phenylindol,
2-[2-(N,N-Dimethylamino)-äthylaminocarbonyl]-3-benzoyloxy-1-phenylindol,
2-[3-(N,N-Diäthylamino)-propylaminocarbonyl]-3-benzoyloxy-1-phenylindol,
2-[4-(N,N-Diäthylamino)-butylaminocarbonyl]-3-benzoyloxy-1-phenylindol,
2-[2-(N,N-Dimethylamino)-äthylaminocarbonyl]-3-pivaloyloxy-1-(2,3-dimethylphenyl)-indol,
2-[3-(N,N-Dimethylamino)-propylaminocarbonyl]-3-pivaloyloxy-1-(4-chlorphenyl)-indol,
2-[2-(N,N-Dimethylamino)-äthylaminocarbonyl]-3-pivaloyloxy-1-(4-chlorphenyl)-indol,
2-[2-(N,N-Diäthylamino)-äthylaminocarbonyl]-3-pivaloyloxy-1-phenyl-5-bromindol,

Die neuen 3-Acyloxy-1-phenyl-2-aminocarbonylindol-Verbindungen der Formel I und deren Säureadditionssalze können erhalten werden, indem man in an sich bekannter Weise Verbindungen der allgemeinen Formel II

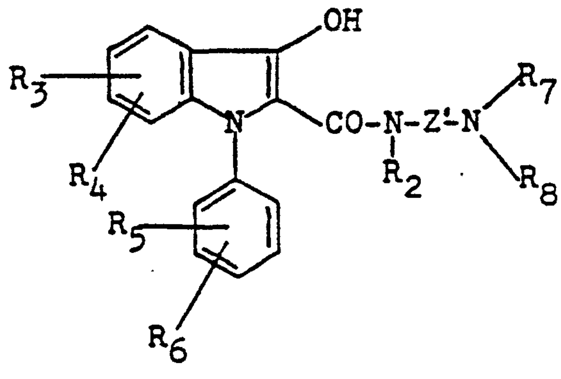

II

worin

$R_2$, $R_3$, $R_4$, $R_5$, $R_6$, $R_7$ und $R_8$ obige Bedeutung besitzen und Z' eine Alkylenkette mit 2 bis 5 Kohlenstoffatomen bedeutet, welche gegebenenfalls an einem nicht an Stickstoff gebundenen Kohlenstoff durch Hydroxy substituiert ist, mit einem Säurederivat der allgemeinen Formel III

$R_1$ - X III

worin

$R_1$ obige Bedeutung besitzt und X eine reaktive Gruppe bedeutet oder, sofern $R_1$ eine freie Carboxylgruppe enthält, auch einem cyclischen Anhydrid einer Säure $R_1OH$ acyliert und gegebenenfalls freie Verbindungen der Formel I in ihre Säureadditionssalze überführt oder die Säureadditionssalze in die freien Verbindungen der Formel I überführt.

Die Umsetzung der Säurederivate der Formel III oder der cyclischen Anhydride mit den Hydroxyverbindungen der Formel II kann nach an sich zur Esterbildung durch Acylierung üblichen Methoden ausgeführt werden. Als reaktionsfähige Derivate kommen insbesondere Säurehalogenide, vorzugsweise -chloride, Ester und Anhydride oder gemischte Anhydride der Säuren $R_1OH$ in Frage, z. B. Verbindungen der Formel III, worin die reaktive Gruppe X Halogen, insbesondere Chlor oder Brom, eine Gruppe $OR_1$, worin $R_1$ obige Bedeutung besitzt, oder eine Gruppe O-CO-W bedeutet, worin W für $C_1$-$C_4$-Alkyl oder $C_1$-$C_4$-Alkoxy steht. Die Acylierung kann in einem unter den Reaktionsbedingungen inerten Lösungsmittel bei Temperaturen zwischen Raumtemperatur und dem Siedepunkt des Lösungsmittels erfolgen. Als Lösungsmittel eignen sich halogenierte Kohlenwasserstoffe wie Methylenchlorid oder Chloroform, aromatische Kohlenwasserstoffe wie Benzol, Toluol, Xylol oder Chlorbenzol, cyclische Äther wie Tetrahydrofuran oder Dioxan, Dimethylformamid oder Gemische dieser Lösungsmittel.

Die Acylierung wird zweckmäßig in Gegenwart eines säurebindenden Reagenzes durchgeführt. Als säurebindende Mittel eignen sich anorganische Basen, insbesondere Alkalimetallcarbonate und -hydroxyde wie z. B. Kaliumcarbonat oder Kaliumhydroxyd oder organische Basen, insbesondere tertiäre Niederalkylamine und Pyridine wie z. B. Triäthylamin oder 4-Dimethylaminopyridin.

Falls die Ausgangsverbindungen außer der zu veresternden Hydroxygruppe in 3-Stellung noch eine weitere freie Hydroxygruppe in dem Seitenkettenglied Z' enthalten, findet unter den vorstehenden Acylierungsbedingungen die Acylierung bevorzugt an der enolischen Hydroxygruppe in 3-Stellung statt. Je nach Menge des eingesetzten Säurederivates und der Reaktionsdauer kann jedoch die Hydroxygruppe in der Seitenkette ganz oder teilweise ebenfalls zu einer $R_1O$-Gruppe acyliert werden. Allfällige Gemische von mono- und diacylierten Verbindungen der Formel I lassen sich auf an Sich bekannte Weise, z. B. durch Chromatographie, trennen.

Zur Herstellung von Verbindungen der Formel I, welche eine freie Hydroxygruppe in dem Seitenkettenglied Z enthalten, kann auch in den entsprechenden Ausgangsverbindungen der Formel II die freie Hydroxygruppe in dem Glied Z' gewünschtenfalls vor der Umsetzung in an sich bekannter Weise mit einer Schutzgruppe versehen sein, welche anschließend leicht wieder abspaltbar ist unter Bedingungen, unter denen die Estergruppe in 3-Stellung nicht angegriffen wird. Beispielsweise eignen sich zum Schutz einer sekundären aliphatischen Hydroxygruppe leicht spaltbare Carbonate wie Benzylcarbonat.

Verbindungen der Formel I, worin Z eine Hydroxy- oder Acyloxygruppe enthält, werden bei der Synthese in Form ihrer Racemate erhalten. Unter den Schutz dieser Erfindung fallen die racemischen Gemische wie auch die optisch aktiven Formen dieser Verbindungen. Die optisch aktiven Verbindungen können aus den racemischen Gemischen in an sich bekannter Weise durch Umsetzung mit geeigneten optisch aktiven Säuren. wie beispielsweise Weinsäure, 0,0'-Dibenzoyl-Weinsäure, Mandelsäure, Di-O-isopropyliden-2-oxo-L-gulonsäure, und anschließende fraktionierte Kristallisation der gewonnenen Salze in ihre optisch aktiven Antipoden aufgetrennt werden.

Die Auftrennung in die optisch aktiven Verbindungen kann gewünschtenfalls auch in einer geeigneten Vorstufe, z. B. den Verbindungen der Formel II, erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden, und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit Chlorwasserstoffsäure, Bromwasserstoffsäure, Schwefelsäure, Phosphorsäure, Methansulfonsäure, Äthansulfonsäure, Cyclohexylaminosulfonsäure, Amidosulfonsäure, Essigsäure, Milchsäure, Weinsäure, Phenylessigsäure oder Mandelsäure. Sofern in dem Rest $R_1$ der Verbindungen der Formel I noch eine freie COOH-Gruppe enthalten ist, können die Verbindungen auch innere Salze bilden.

Die Ausgangsverbindungen der Formel II können erhalten werden, indem man aus Verbindungen der Formel IV

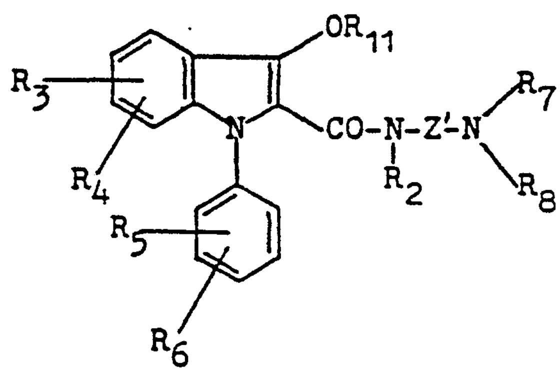

IV

worin $R_2$, $R_3$, $R_4$, $R_5$, $R_6$, $R_7$, $R_8$ und Z' obige Bedeutung besitzen und $R_{11}$ Methyl oder eine hydrogenolytisch abspaltbare Gruppe, z. B. eine gegebenenfalls substituierte Benzylgruppe, bedeutet, die Hydroxygruppe in 3-Stellung auf an sich bekannte Weise freisetzt.

Die Spaltung der $OR_{11}$-Gruppe kann nach an sich zur Ätherspaltung üblichen Methoden erfolgen. Benzyläther lassen sich z. B. hydrogenolytisch spalten.

Verbindungen der Formel II können auch erhalten werden, indem man Verbindungen der Formel X

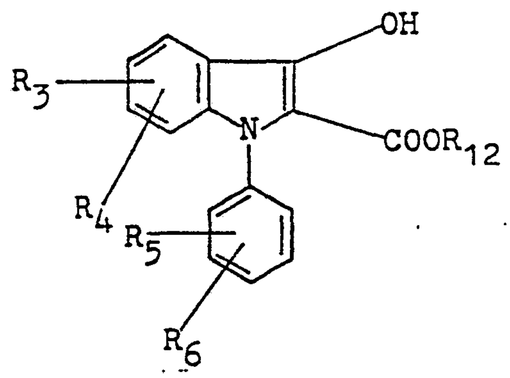

X

worin $R_3$, $R_4$, $R_5$ und $R_6$ obige Bedeutung besitzen und $R_{12}$ niederes Alkyl bedeutet, mit Verbindungen der Formel VI,

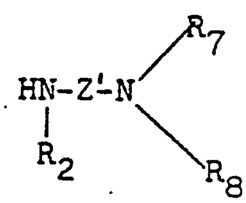

VI

worin $R_2$, $R_7$, $R_8$ und Z' obige Bedeutung besitzen, umsetzt. Die Umsetzung kann auf an sich zur Esteraminolyse bekannte Weise erfolgen. z. B. kann die Umsetzung in einem unter den Reaktionsbedingungen inerten Lösungsmittel bei Temperaturen zwischen Raumtemperatur und dem Siedepunkt des Lösungsmittels in an sich zur Bildung von Amid-Gruppierungen durch Aminoacylierung üblicher Weise durchgeführt werden.

Die Ausgangsverbindungen der Formel IV können auf an sich bekannte Weise hergestellt werden, indem man Verbindungen der Formel V

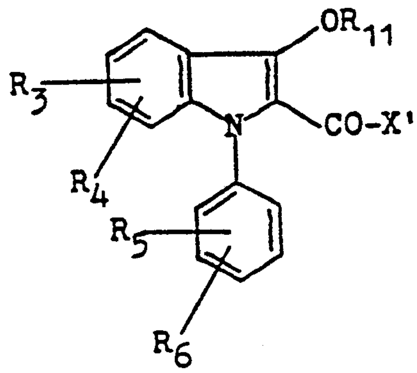

V

worin $R_3$, $R_4$, $R_5$, $R_6$, $R_{11}$ obige Bedeutung besitzen, und X' eine reaktive Gruppe bedeutet, mit einer Verbindung der Formel VI umsetzt. Die Umsetzung kann nach zur Bildung von Amidgruppen durch Aminoacylierung üblichen Methoden auf an sich bekannte Weise erfolgen. Als reaktive Gruppe X' kommen Halogen, insbesondere Chlor oder Brom, Alkoxy mit 1 bis 4 Kohlenstoffatomen oder eine Gruppe O-CO-W, worin W für $C_1$-$C_4$-Alkyl oder $C_1$-$C_4$-Alkoxy steht, in Frage.

Verbindungen der Formel Va

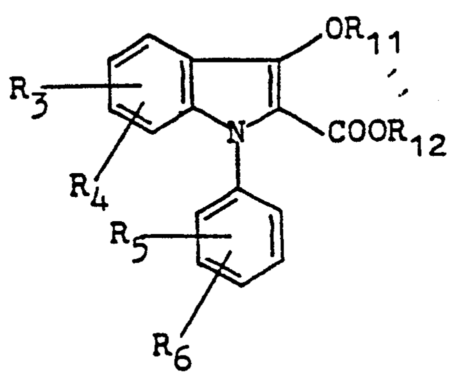

Va

worin $R_3$, $R_4$ $R_5$, $R_6$, $R_{11}$ und $R_{12}$ obige Bedeutung besitzen, können auf an sich bekannte Weise erhalten werden, indem man

a') Verbindungen der Formel VII

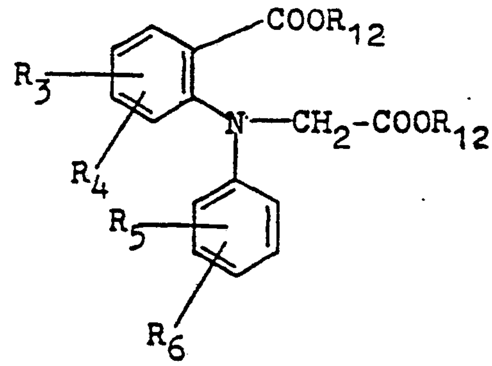

VII

worin $R_3$, $R_4$, $R_5$, $R_6$ und $R_{12}$ obige Bedeutung besitzen, in an sich bekannter Weise cyclisiert zu Verbindungen der Formel X und diese auf an sich bekannte Weise, z. B. nach zur Verätherung üblichen Methoden, in Verbindungen der Formel Va überführt oder

b') zur Herstellung von Verbindungen der Formel Va Verbindungen der Formel VIII

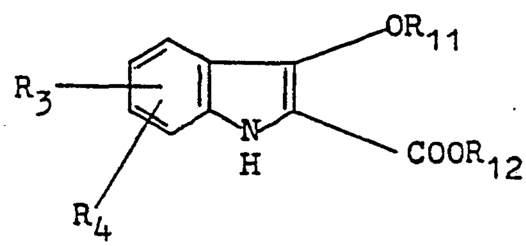

VIII

worin $R_3$, $R_4$, $R_{11}$ und $R_{12}$ obige Bedeutung besitzen, mit Verbindungen der Formel IX

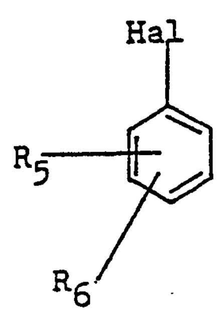

IX

worin $R_5$ und $R_6$ obige Bedeutung besitzen und Hal Halogen bedeutet, umsetzt.

Die Verbindungen der Formel Va können anschließend auf an sich bekannte Weise zu den entsprechenden Säuren hydrolysiert und diese in an sich bekannter Weise in weitere reaktionsfähige Säurederivate überführt werden. Die Überführung der freien Säuren in reaktive Säurederivate erfolgt ebenfalls auf an sich bekannte Weise. So können Säurehalogenide der Formel V zum Beispiel durch Umsetzung der Säuren mit einem anorganischen Säurehalogenid erhalten werden. Gemischte Säureanhydride können zum Beispiel durch Umsetzung von Alkalimetallsalzen der Säuren mit einem entsprechenden organischen Säurechlorid in Gegenwart einer tertiären organischen Base erhalten werden.

Die Cyclisierung der Verbindungen der Formel VII gemäß Verfahrensvariante a') wird zweckmäßigerweise in einem unter den Reaktionsbedingungen inerten Lösungsmittel in Gegenwart einer starken Base bei erhöhter

Temperatur, beispielsweise bei Temperaturen zwischen 50 und 150 °C, durchgeführt. Als starke Basen eignen sich beispielsweise niedere Alkalimetallalkoholate wie z. B. Natriummethylat. Als inerte Lösungsmittel eignen sich beispielsweise die entsprechenden niederen Alkohole, aromatische Kohlenwasserstoffe wie Toluol oder Xylol, oder Mischungen solcher Lösungsmittel. Bei der Umsetzung fällt die Verbindung der Formel X in Form ihres Alkalimetallsalzes an und kann bei der Aufarbeitung durch Ansäuern des Reaktionsgemisches in Freiheit gesetzt werden.

Die für die Verfahrenvariante a') benötigten Ausgangsverbindungen der Formel VII können auf an sich bekannte Weise erhalten werden ausgehend von Phenylglycinverbindungen der Formel XI

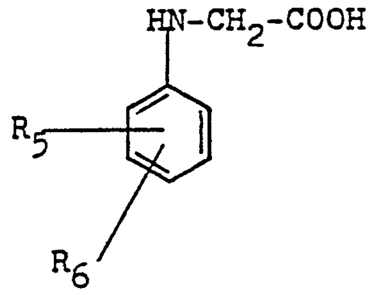

XI

worin $R_5$ und $R_6$ obige Bedeutung besitzen, und welche durch Umsetzung der entsprechenden Aniline mit Chloressigsäure erhalten werden können, und o-Chlorbenzoesäuren der Formel XII

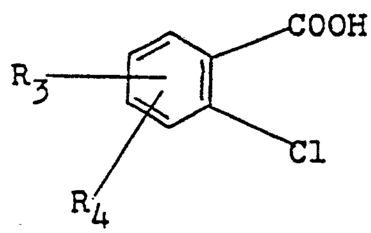

XII

worin $R_3$ und $R_4$ obige Bedeutung besitzen. Die Alkalimetallsalze, insbesondere Kaliumsalze, der Säuren der Formel XI werden mit Alkalimetallsalzen, insbesondere Kaliumsalzen, der Säuren der Formel XII bei erhöhter Temperatur, beispielsweise Temperaturen zwischen 100 und 150°C, in Gegenwart einer anorganischen Base, beispielsweise Kaliumcarbonat, und eines Kupferkatalysators, beispielsweise Kupferpulver, in einem polaren Lösungsmittel, vorzugsweise Wasser oder einer Mischung aus Wasser und einem mit Wasser mischbaren organischen Lösungsmittel, zu Verbindungen der Formel XIII

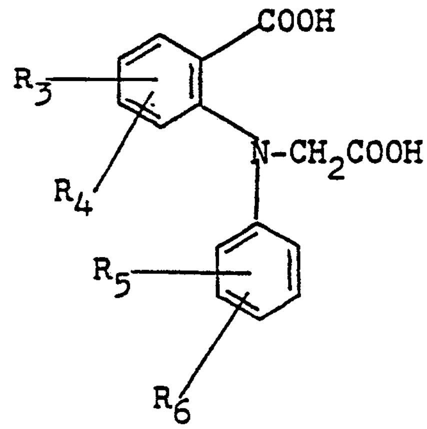

XIII

worin $R_3$, $R_4$, $R_5$ und $R_6$ obige Bedeutung besitzen, umgesetzt, und diese dann in an sich bekannter Weise zu Verbindungen der Formel VII verestert, beispielsweise durch Umsetzung mit einem Alkohol $R_{12}OH$ in Gegenwart von Schwefelsäure bei erhöhter Temperatur, beispielsweise Siedetemperatur des Reaktionsgemisches.

Die vorstehend beschriebene Verfahrensvariante a') ist insbesondere zur Herstellung solcher Verbindungen der Formel Va, worin $R_3$ und $R_4$ für Wasserstoff oder für solche Substituenten stehen, welche nicht zu einer Umsetzung mit Phenylglycin befähigt sind. Falls die Verbindung der Formel XIII weitere zur Umsetzung mit Phenylglycin befähigte Substituenten enthält, können bei dieser Umsetzung neben der Verbindung der Formel XIII auch mehrfach substituierte Nebenprodukte in dem Reaktionsgemisch entstehen. Das gewünschte Reaktionsprodukt kann auf chromatographischem Wege von allfälligen Nebenprodukten abgetrennt werden.

Die Umsetzung von Verbindungen der Formel VIII mit Verbindungen der Formel IX gemäß Verfahrensvariante b') kann auf an sich bekannte Weise erfolgen. Zweckmäßigerweise werden die Verbindungen der Formel VIII in Form ihrer Alkalimetallsalze, beispielsweise ihrer Natrium- oder Lithiumsalze, mit Verbindungen der Formel IX in einem unter den Reaktionsbedingungen inerten Lösungsmittel bei Temperaturen zwischen ca. 100 und 170°C umgesetzt. Als Lösungsmittel eignen sich in dem angegebenen Temperaturbereich siedende inerte organische Lösungsmittel, vorzugsweise Dimethylformamid. Es ist zweckmäßig, die Reaktion in Gegenwart eines Kupferkatalysators, beispielsweise Kupferpulver oder Kupfer-I oder Kupfer-II-Halogeniden, durchzuführen. Die Alkalimetallsalze der Verbindungen der Formel VIII können auf an sich bekannte Weise durch Umsetzen mit einer starken Base, beispielsweise einem Alkalimetallalkoholat oder -hydrid oder -hydroxid, gewünschtenfalls in situ hergestellt werden.

Die für die Verfahrensvariante b') benötigten Ausgangsverbindungen der Formel VIII können auf an sich bekannte Weise ausgehend von Anthranilsäureestern der Formel XIV

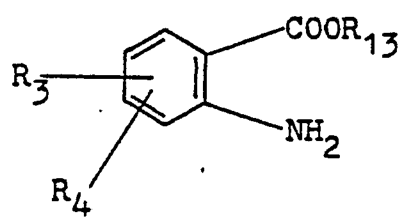

XIV

worin $R_3$ und $R_4$ obige Bedeutung besitzen und $R_{13}$ niederes Alkyl bedeutet, hergestellt werden, indem man diese zunächst mit Chloressigsäure oder einem Chloressigsäureniederalkylester in an sich bekannter Weise zu Verbindungen der Formel XV

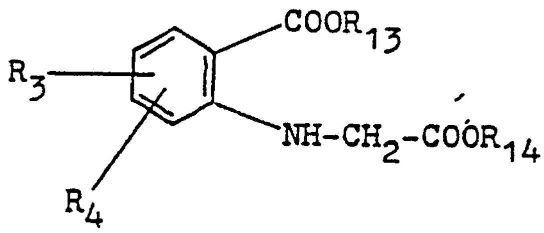

XV

worin $R_3$, $R_4$ und $R_{13}$ obige Bedeutung besitzen und $R_{14}$ Wasserstoff oder niederes Alkyl bedeutet, umsetzt, falls $R_{14}$ Wasserstoff ist, diese Säure in an sich bekannter Weise mit einem Alkohol $R_{12}OH$, worin $R_{12}$ obige Bedeutung besitzt, verestert, und die erhaltenen Diester in an sich bekannter Weise zu Verbindungen der Formel XVI

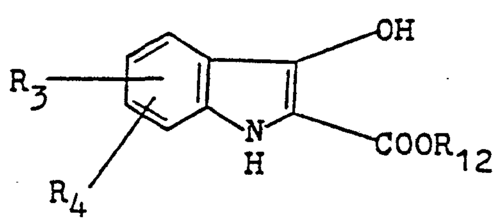

XVI

worin $R_3$, $R_4$ und $R_{12}$ obige Bedeutung besitzen, cyclisiert, welche dann durch Verätherung der Hydroxygruppe auf an sich bekannte Weise in Verbindungen der Formel VIII überführt werden. Die Cyclisierung der Ester der Formel XV kann beispielsweise unter den für die Cyclisierung der Ester der Formel VII vorstehend beschriebenen Bedingungen erfolgen.

Die Verfahrensvariante b') eignet sich insbesondere zur Herstellung solcher Verbindungen der Formel Vb, worin $R_5$ und $R_6$ für Wasserstoff oder für solche Substituenten stehen, welche nicht zu einer Umsetzung mit den Alkalimetallsalzen der Verbindungen der Formel XIII befähigt sind.

Die Verbindungen der Formel I und ihre pharmakologisch akzeptablen Säureadditionssalze zeichnen sich durch interessante pharmakologische Eigenschaften aus und zeigen insbesondere herzrhythmisierende Wirkungen. Die neuen Verbindungen zeichnen sich aus durch eine gute Wirksamkeit und hohe physiologische Verträglichkeit. So zeigen die neuen Verbindungen schon in geringen Dosen eine befriedigende antiarrhythmische Wirkung. Darüber hinaus ist eine unerwünschte negative Beeinflussung des Kontraktionsvermögens des Herzens äußerst gering. Das heißt, die Verbindungen weisen ein besonders günstiges Verhältnis von antiarrhythmischen, bzw. die Refraktärzeit des Herzens verlängernden Wirkungen zu negativ inotropen Nebenwirkungen auf und besitzen eine große therapeutische Breite.

Die zu verwendenden Dosen variieren naturgemäß je nach Art der verwendeten Substanz, der Applikationsart und des zu behandelnden Zustandes. Im allgemeinen werden jedoch befriedigende Ergebnisse in Tierversuchen mit Dosen von 0,1 bis 100 mg/kg Körpergewicht erhalten.

Die antiarrhythmischen Wirkungen der Verbindungen lassen sich in pharmakologischen Standardtestmethoden nachweisen.

Ein günstiges Verhältnis von die funktionelle Refraktärzeit verlängernder Wirkung (FRZ) zu die Kontraktionskraft vermindernder Wirkung (Kraft) der Verbindungen läßt sich zum Beispiel am isolierten linken Vorhof von weiblichen Meerschweinchen von 300 bis 400 g Körpergewicht gemäß der Doppelreizmethode von Govier [J.Pharmakol. Exp. Ther. 148 (1965) 100-105] zeigen. In der nachfolgenden Tabelle sind angegeben diejenige Konzentration in µmol/l, bei der es 18 Minuten nach Applikation zu einer Verlängerung der funktionellen Refraktärzeit auf 125 % kommt und diejenige Konzentration, bei der es zu einer Verminderung der Kontraktionskraft auf 75 % des Ausgangswertes kommt, und außerdem der Quotient aus diesen Konzentrationen, welcher ein Indiz für die therapeutische Breite der Verbindungen darstellt.

| Beispiel Nr. | isolierter Meerschweinchen-Vorhof Kraft [µmol/l] | FRZ [µmol/l] | Kraft / FRZ |
|---|---|---|---|
| 3 | 4,2 | 3,0 | 1,4 |
| 2 | 6,2 | 2,8 | 2,2 |
| 20 | 1,8 | 0,73 | 2,5 |

Ebenso zeigen die Verbindungen in Ratten in der Versuchsmethode nach Raschak (Arzneimittelforsch. 25 (1975) 639-641) eine hemmende Wirkung gegenüber den durch Aconitin-Infusion induzierten Herzrhythmusstörungen.

Aufgrund der vorstehend beschriebenen Wirkungen eignen sich die Verbindungen der Formel I und ihre pharmakologisch akzeptablen Säureadditionssalze als Arzneimittel zur Behandlung von Herzrhythmusstörungen.

Ferner zeigen die Substanzen der Formel I auch antithrombotische Eigenschaften.

Als Heilmittel können die Verbindungen der Formel I und ihre physiologisch verträglichen Säureadditionssalze mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z. B. Milchzucker, Stärke oder Talkum oder flüssiger Verdünnungsmittel wie z. B. Wasser, fetten Ölen oder flüssigen Paraffinen.

Die nachfolgenden Beispiele sollen die Herstellung der neuen Verbindungen der Formel I näher erläutern jedoch den Umfang der Erfindung in keiner Weise beschränken.

**Beispiel 1:**

2-[3-(N N-Diäthylamino)propylaminocarbonyl]-3-acetoxy-1-phenylindol.

A) 475 g Chloressigsäure (5 Mol) werden mit 930 g Anilin (10 Mol) in 2 l Wasser 1,5 Stunden lang auf 100°C erhitzt. Nach dem Abkühlen wird das gebildete N-Phenylglycin abgesaugt und mit Wasser gewaschen. Ausbeute 468 g (= 62 % bezogen auf Chloressigsäure).

B) 468 g N-Phenylglycin werden unter Erwärmung in 1,3 l Methanol gelöst. Die Lösung wird unter Kühlung mit einer Lösung von 205 g Kaliumhydroxyd in 450 ml Methanol versetzt. Nach dem Abkühlen wird das ausgefallene Kaliumsalz des N-Phenylglycins abgesaugt. Ausbeute 392 g = 67 %.

C) 468 g o-Chlorbenzoesäure werden unter Erwärmung in 1,5 l Isopropanol gelöst. Die Lösung wird unter Kühlung mit einer Lösung von 198 g Kaliumhydroxyd in 200 ml Methanol versetzt. Nach dem Abkühlen wird das ausgefallene Kaliumsalz der o-Chlorbenzoesäure abgesaugt. Ausbeute 394 g = 67,4 %.

D) 750 g des Kaliumsalzes des N-Phenylglycins werden mit 808 g des Kaliumsalzes der o-Chlorbenzoesäure, 268 g Kaliumcarbonat und 1,5 g Kupfer-Pulver in 385 ml Wasser 5 Stunden lang auf 120 bis 125°C (Innentemperatur) erhitzt. Nach dem Lösen des Reaktionsgemisches in Wasser wird die Lösung mit Salzsäure angesäuert und die ausgefallene N-Diphenylglycin-o-carbonsäure abgesaugt. Ausbeute 675 g = 62,7 %.

E) 675 g N-Diphenylglycin-o-carbonsäure werden mit 2,5 l Methanol und 500 ml Schwefelsäure 5 Stunden lang zum Sieden erhitzt. Das Methanol wird teilweise abgedampft, die Reaktionsmischung anschließend in Wasser gegossen und mit Methylenchlorid extrahiert. Die Methylenchloridphase wird mit Sodalösung ausgeschüttelt, getrocknet und eingedampft, wobei der rohe N-Diphenylglycin-o-carbonsäuredimethylester als Rückstand erhalten wird. Ausbeute 614 g Rohprodukt = 82,4 %.

F) 47,1 g Natrium werden in 500 ml Methanol gelöst und die Lösung mit 500 ml Toluol versetzt. Das Gemisch wird zum Sieden erhitzt und unter schwachem Rückfluß mit einer Lösung von 614 g N-Diphenylglycin-o-carbonsäuredimethylester in 1,5 l Toluol versetzt. Nach weiteren 30 Minuten Siedens wird die Reaktionsmischung abgekühlt und in 1 l Wasser gegeben und mit 250 ml Salzsäure angesäuert. Der ausgefallene N-Phenylindoxylsäuremethylester wird abgesaugt. Ausbeute 464 g = 84,8 %.

G) 140 g N-Phenylindoxylsäuremethylester werden in 600 ml Aceton mit 69 ml Dimethylsulfat und 71 g Kaliumcarbonat während 4 Stunden unter Rühren zum Sieden erhitzt. Die Reaktionsmischung wird in Wasser gegossen und der gebildete N-Phenyl-3-methoxyindol-2-carbonsäuremethylester wird abgesaugt und in 450 ml Methanol aufgelöst. Die Lösung wird mit einer Lösung von 42 g Natriumhydroxyd in 50 ml Wasser versetzt und 30 Minuten lang zum Sieden erhitzt. Das Reaktionsgemisch wird in Wasser gelöst, die wäßrige Lösung wird mit Salzsäure angesäuert und die ausgefallene N-Phenyl-3-methoxyindol-2-carbon-säure wird abgesaugt. Ausbeute 127 = 90.7 %.

H) 92 g N-Phenyl-3-methoxyindol-2-carbonsäure werden in 920 ml Äther und 30,3 g Pyridin gelöst. Die Lösung wird unter Eiskühlung in eine Lösung von 28,3 ml Thionylchlorid in 160 ml Äther unter Rühren eingetropft. Die Mischung wird 1 Stunde lang bei Raumtemperatur gerührt und die ausgefallenen Pyridinsalze werden abgesaugt.

Die erhaltene ätherische Lösung des N-Phenyl-3-methoxyindol-2-carbonsäurechlorids wird unter Eiskühlung zu einer Lösung von 550 g 1-Amino-3-diäthylaminopropan und 40 g Triäthylamin in 70 ml Methylenchlorid getropft. Das Reaktionsgemisch wird 1 Stunde bei Raumtemperatur gerührt und anschließend mit verdunnter Salzsäure extrahiert. Der Salzsäureextrakt wird durch Zugeben von Natronlauge alkalisch gemacht und mit Äther extrahiert. Die Ätherlösung wird mit Wasser gewaschen, über Natriumsulfat getrocknet und eingedampft. Es verbleibt 2-[3-(N,N-Diäthylamino)propylaminocarbonyl]-3-methoxy-1-phenylindol als ölige Base. Diese wird in Isopropanol gelöst und in ihr Hydrochlorid überführt. Schmelzpunkt 135-137° C.

I) 2,1 g 2-[3-(N,N-Diäthylamino)propylaminocarbonyl]-3-methoxy-1-phenylindol-hydrochlorid werden in 5 ml Methanol gelöst und mit 2,5 ml konzentrierter Salzsäure versetzt. Das Reaktionsgemisch wird 3 Stunden lang unter Rückfluß gekocht und anschließend im Vakuum eingedampft. Man erhält 1,66 g (82 % der Theorie) 2-[3-(N,N-Diäthylamino)propylaminocarbonyl]-3-hydroxy-1-phenyl-indol-hydrochlorid als ölige Substanz. IR: 1647 $cm^{-1}$ (CO Amid).

J) 1,66 g 2-[3-(N,N-Diäthylamino)propylaminocarbonyl]-3-hydroxy-1-phenylindol-hydrochlorid werden in 25 ml Dichlormethan gelöst und nacheinander unter kräftigem Rühren mit 0,6 ml Triäthylamin und 0,3 ml Acetylchlorid versetzt. Die Reaktionsmischung wird 30 Minuten lang unter Rückfluß gekocht. Nach Abkühlen wird die Mischung mit wenig gesättigter Kochsalzlösung gewaschen, die organische Phase über Natriumsulfat getrocknet und im Vakuum eingedampft. Nach Umkristallisieren aus Isopropanol erhält man 1,00 g (57 % der Theorie) 2-[3-(N,N-Di-äthylamino)-propylaminocarbonyl]-3-acetoxy-1-phenylindol-hydrochlorid. Fp.: 174-176° C.

IR-Spektrum: 1771 $cm^{-1}$, 1663 $cm^{-1}$

**Beispiel 2:**

2-[3-(N,N-Diäthylamino)-2-hydroxypropylaminocarbonyl]-3-pivaloyloxy-1-phenylindol.

A) Die aus Umsetzung von 92 g N-Phenyl-3-methoxyindol-2-carbonsäure mit Thionylchlorid und Pyridin in Äther gemäß Beispiel 1H erhaltene ätherische Lösung von N-Phenyl-3-methoxy-indol-2-carbonsäurechlorid wird unter Eiskühlung zu einer Lösung von 579 g 1-Amino-2-hy-droxy-3-diäthylaminopropan und 40 g Triäthylamin in 70 ml Methylenchlorid getropft. Das Reaktionsgemisch wird 1 Stunde bei Raumtemperatur gerührt und anschließend wie in Beispiel 1H beschrieben augearbeitet. Es werden 110 g 2-[3-(N,N-Diäthylamino)-2-hydroxypropylaminocarbonyl]-3-methoxy-1-phenylindol-hydrochlorid erhalten, Schmelzpunkt 148-150° C.

B) 15 g 2-[3-(N,N-Diäthylamino)-2-hydroxypropylaminocarbonyl]-3-methoxy-1-phenylindol-hydrochlorid werden mit 15 ml Methanol und 15 ml konzentrierter Salzsäure 3 Stunden lang unter Rückfluß gekocht. Anschließend wird das Reaktionsgemisch im Vakuum eingedampft. Das als Rückstand verbleibende 2-[3-(N,N-Diäthylamino)-2-hydroxypropylaminocarbonyl]-3-hydroxy-1-phenylindolhydrochlorid besitzt nach Umkristallisieren aus Isopropanol einen Schmelzpunkt von 166-168° C.

C) 12,6 g 2-[3-(N,N-Diäthylamino)-2-hydroxypropylaminocarbonyl]-3-hydroxy-1-phenylindol-hydrochlorid (0,03 Mol) werden mit 150 ml Methylenchlorid, 4,2 ml Triäthylamin (0,03 Mol) und 3,9 ml Pivaloylchlorid (0,03 Mol) versetzt und das Reaktionsgemisch wird 3 Stunden bei Raumtemperatur stehengelassen. Anschließend wird mit gesättigter Kochsalzlösung gewaschen und über Natriumsulfat getrocknet und filtriert. Die Lösung wird sodann eingedampft, der Rückstand in Methylenchlorid gelöst und durch Niederdruckchromatographie über eine Kieselgelsäure gereinigt. Als Elutionsmittel wird zunächst Methylenchlorid und anschließend 10 % Methanol enthaltendes Methylenchlorid verwendet. Das nach Eindampfen des Eluates als Rückstand erhaltene 2-[3-(N,N-Diäthylamino)-2-hydroxypropylaminocarbonyl]-3-pivaloyloxy-1-phenylindol-hydrochlorid wird anschließend aus Isopropanol umkristallisiert, Schmelzpunkt 157-159° C.

IR-Spektrum: 1758 $cm^{-1}$, 1661 $cm^{-1}$.

**Beispiel 3:**

2-[3-(N,N-Diäthylamino)-2-hydroxypropylaminocarbonyl]-3-benzoyloxy-1-phenylindol.

2,1 g 2-[3-(N,N-Diäthylamino)-2-hydroxypropylaminocarbonyl]-3-hydroxy-1-phenylindol-hydrochlorid (0,005 Mol, hergestellt analog Beispiel 2 B) werden in 25 ml trockenem Methylenchlorid mit 0,3 g (0,01 Mol) Natriumhydrid (80 %-ig) versetzt. Nach 30 Minuten werden in der Kälte 1,5 ml Benzoylchlorid (0,012 Mol) zugesetzt und das Reaktionsgemisch 16 Stunden bei Raumtemperatur stehengelassen. Anschließend wird die Lösung filtriert, mit gesättigter Kochsalzlösung gewaschen, über Natriumsulfat getrocknet und eingedampft. Das als Rückstand erhaltene Rohprodukt besteht aus einem Gemisch der Hydrochloride des 2-[3-(N,N-Diäthylamino)-2-hydroxypropylaminocarbonyl]-3-benzoyloxy-1-phenylindol und des 2-[3-(N,N-Diäthylamino)-2-benzoyloxypropylaminocarbonyl]-3-benzoyloxy-1-phenylindol. Dieses Rohprodukt wird in Äthylacetat gelöst und die Lösung mit Diäthyläther versetzt, wobei das Hydrochlorid des 2-[3-(N,N-Diäthylamino)-2-hydroxypropylaminocarbonyl]-3-benzoyloxy-1-phenylindol kristallin erhalten wird. Nach Umkristallisation aus Äthylacetat/Diäthyläther zeigt es einen Schmelzpunkt von 130-133°C
IR-Spektrum: 1735 $cm^{-1}$, 1654 $cm^{-1}$.

**Beispiel 4:**

2-[3-(N,N-Diäthylamino)-2-acetoxypropylaminocarbonyl]-3-acetoxy-1-phenylindol.

10,5 g 2-[3-(N,N-Diäthylamino)-2-hydroxypropylaminocarbonyl]-3-hydroxy-1-phenylindol-hydrochlorid (0,025 Mol) werden mit 100 ml Methylenchlorid und 7,0 ml Triäthylamin (0,05 Mol) versetzt. Anschließend werden der Reaktionslösung 3,6 ml Acetylchlorid (0,05 Mol) zugefügt und das Reaktionsgemisch 18 Stunden bei Raumtemperatur stehengelassen. Dann wird die Lösung mit gesättigter Kochsalzlösung gewaschen, über Natriumsulfat getrocknet, filtriert und eingedampft. Das als Rückstand verbleibende Rohprodukt wird durch Niederdruckchromatographie wie in Beispiel 2 beschrieben gereinigt. Man erhält das 2-[3-(N,N-Diäthylamino)-2-acetoxypropylaminocarbonyl]-3-acetoxy-1-phenylindol-hydrochlorid als öliges Produkt.
IR-Spektrum: 1778 $cm^{-1}$, 1741 $cm^{-1}$, 1663 $cm^{-1}$.

**Beispiel 5**

2-[3-(N,N-Diäthylamino)-2-hydroxypropylaminocarbonyl]-3-methoxycarbonyläthylcarbonyloxy-1-phenylindol.

2,1 g 2-[3-(N,N-Diäthylamino)-2-hydroxypropylaminocarbonyl]-3-hydroxy-1-phenylindol-hydrochlorid (0,005 Mol) werden in 25 ml Methylenchlorid mit 0,7 ml Triäthylamin (0,005 Mol) und 0,6 ml Bernsteinsäuremethylesterchlorid versetzt und das Reaktionsgemisch 1 Stunde bei Raumtemperatur gerührt. Anschließend wird die Lösung mit gesättigter Kochsalzlösung gewaschen, über Natriumsulfat getrocknet, filtriert und eingedampft. Der Rückstand wird durch Niederdruckchromatographie unter Verwendung von Methylenchlorid als Elutionsmittel wie in Beispiel 2 beschrieben gereinigt. Man erhält das 2-[3-(N,N-Diäthylamino)-2-hydroxypropylaminocarbonyl]-3-methoxycarbonyläthylcarbonyloxy-1-phenylindol-hydrochlorid als öliges Produkt.
IR-Spektrum: 1768 $cm^{-1}$, 1734 $cm^{-1}$, 1654 $cm^{-1}$.

Nach den in den Beispielen 1 bis 5 beschriebenen Verfahren können auch die in der folgenden Tabelle aufgeführten Verbindungen der Formel I durch Acylierung entsprechender Verbindungen der Formel II bzw. Amidbildung aus Verbindungen der Formel Va hergestellt werden.

| Beispiel Nr. | $R_3$ | $R_4$ | $R_5$ | $R_6$ | $R_2$ | Z | $R_7$ | $R_8$ | $R_1$ | Bemerkungen<br>Fp in °C, HCl = Hydrochlorid<br>IR-banden in $cm^{-1}$ |
|---|---|---|---|---|---|---|---|---|---|---|
| 6 | H | H | H | H | H | $CH_2-CH_2-CH_2$ | $C_2H_5$ | $C_2H_5$ | -CO-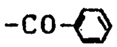 | HCl, Fp.190-192<br>IR: 1736, 1662 |
| 7 | H | H | 4-Cl | H | H | $CH_2-CH_2$ | $CH_3$ | $CH_3$ | $-CO-C(CH_3)_3$ | HCl, Fp.215-217<br>IR: 1761, 1663 |
| 8 | H | H | 4-Cl | H | H | $CH_2-CH_2-CH_2$ | $CH_3$ | $CH_3$ | $-CO-C(CH_3)_3$ | HCl, ölig<br>IR: 1751, 1653 |
| 9 | H | H | 2,3-di-$CH_3$ | | H | $CH_2-CH_2$ | $CH_3$ | $CH_3$ | $-CO-C(CH_3)_3$ | HCl, Fp.118-120<br>IR: 1752, 1663 |
| 10 | 5-Br | H | H | H | H | $CH_2-CH_2$ | $CH_3$ | $CH_3$ | $-CO-C(CH_3)_3$ | HCl, Fp.210-212<br>IR: 1758, 1652 |
| 11 | H | H | 4-Cl | H | H | $CH_2-CH-CH_2$<br>OH | $C_2H_5$ | $C_2H_5$ | $-CO-C(CH_3)_3$ | HCl, ölig<br>IR: 1752, 1663 |
| 12 | H | H | 4-$CH_3$O | H | H | $CH_2-CH-CH_2$<br>OH | $C_2H_5$ | $C_2H_5$ | $-CO-C(CH_3)_3$ | HCl, ölig<br>IR: 1757, 1653 |
| 13 | H | H | 4-F | H | H | $CH_2-CH-CH_2$<br>OH | $C_2H_5$ | $C_2H_5$ | $-CO-C(CH_3)_3$ | HCl, Fp.183-185<br>IR: 1756, 1663 |
| 14 | H | H | 4-F | H | H | $CH_2-CH-CH_2$<br>OH | $C_2H_5$ | $C_2H_5$ | -CO-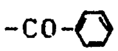 | HCl, Fp.186-188<br>IR: 1741, 1662 |
| 15 | H | H | 4-Cl | H | H | $CH_2-CH-CH_2$<br>OH | $C_2H_5$ | $C_2H_5$ | -CO-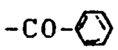 | HCl, Fp.190-194<br>IR: 1743, 1658 |
| 16 | H | H | H | H | H | $CH_2-CH-CH_2$<br>OH | $C_2H_5$ | $C_2H_5$ | -CO-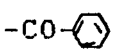-$NO_2$ | HCl, ölig<br>IR: 1749, 1653 |
| 17 | H | H | H | H | H | $CH_2-CH-CH_2$<br>OH | $C_2H_5$ | $C_2H_5$ | -CO-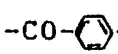-$CH_3$ | HCl, ölig<br>IR: 1734, 1653 |
| 18 | H | H | H | H | H | $CH_2-CH-CH_2$<br>OH | $C_2H_5$ | $C_2H_5$ | -CO-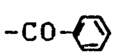-$OCH_3$ | HCl, ölig<br>IR: 1734, 1654 |
| 19 | H | H | H | H | H | $CH_2-CH-CH_2$<br>OH | $C_2H_5$ | $C_2H_5$ | $-CO-n-C_{11}H_{23}$ | HCl, ölig<br>IR: 1765, 1653 |
| 20 | H | H | H | H | H | $CH_2-CH-CH_2$<br>O-CO-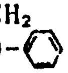 | $C_2H_5$ | $C_2H_5$ | -CO-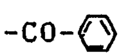 | HCl, Fp.173-175<br>IR: 1744, 1725, 1669 |
| 21 | H | H | H | H | H | $CH_2-CH-CH_2$<br>O<br>CO-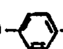-$CH_3$ | $C_2H_5$ | $C_2H_5$ | -CO-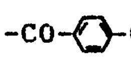-$CH_3$ | HCl, ölig<br>IR: 1744, 1717, 1669 |
| 22 | H | H | 4-Cl | H | H | $CH_2-CH-CH_2$<br>O-CO-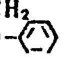 | $C_2H_5$ | $C_2H_5$ | -CO-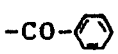 | HCl, Fp.127-129<br>IR: 1741, 1720, 1669 |

**Beispiel I**: Tabletten

Man stellt Tabletten in folgender Zusammensetzung pro Tablette her:

| | |
|---|---|
| 2-[3-(N,N-Diäthylamino)-2-hydroxypropylaminocarbonyl]-3-pivalo-yloxy-1-phenylindol-hydrochlorid | 15 mg |
| Maisstärke | 60 mg |
| Milchzucker | 140 mg |
| Gelatine (als 10 %-ige Lösung) | 6 mg |

Der Wirkstoff, die Maisstärke und der Milchzucker werden mit der 10 %-igen Gelatine-Lösung eingedickt. Die Paste wird zerkleinert und das entstandene Granulat wird auf ein geeignetes Blech gebracht und bei 45°C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

**Patentansprüche**

1. 1-Phenyl-2-aminocarbonylindol-Verbindungen der allgemeinen Formel I

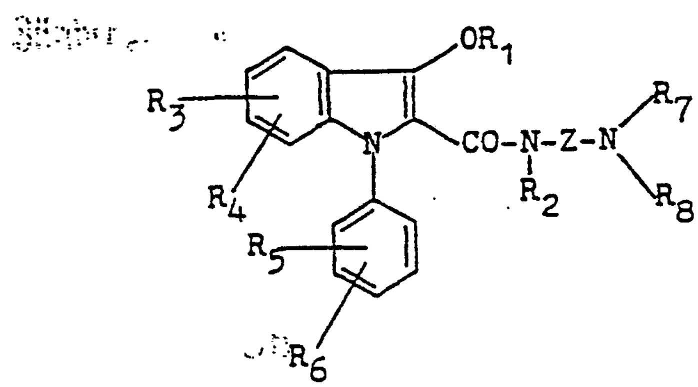

I

worin
$R_1$ eine Alkanoylgruppe mit 2 bis 12 Kohlenstoffatomen bedeutet, welche gegebenenfalls substituiert sein kann durch einen Alkoxyrest mit 1 bis 4 Kohlenstoffatomen oder eine Carboxyl- oder Alkoxycarbonylgruppe, wobei die Alkoxycarbonylgruppe einen Alkoxyrest mit 1 bis 4 Kohlenstoffatomen besitzt, oder
$R_1$ eine Gruppe a

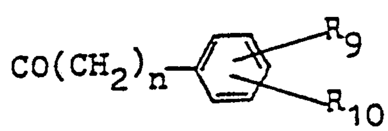

a

bedeutet, worin n 0, 1, 2 oder 3 ist und $R_9$ und $R_{10}$ unabhängig voneinander Wasserstoff, Halogen, Alkyl mit 1 bis 4 Kohlenstoffatomen oder Alkoxy mit 1 bis 4 Kohlenstoffatomen oder falls sie an 2 benachbarte Kohlenstoffatome gebunden sind, auch gemeinsam Methylendioxy darstellen, oder, falls n 0 ist und $R_9$ Wasserstoff darstellt, $R_{10}$ auch Nitro oder Trifluormethyl bedeutet,

$R_2$ Wasserstoff oder Alkyl mit 1 bis 4 Kohlenstoffatomen bedeutet,
$R_3$ Wasserstoff, Halogen, Alkyl mit 1 bis 4 Kohlenstoffatomen oder Alkoxy mit 1 bis 4 Kohlenstoffatomen bedeutet,
$R_4$ Wasserstoff, Halogen, Alkyl mit 1 bis 4 Kohlenstoffatomen oder Alkoxy mit 1 bis 4 Kohlenstoffatomen bedeutet,
$R_5$ Wasserstoff, Alkyl mit 1 bis 4 Kohlenstoffatomen, Halogen oder Alkoxy mit 1 bis 4 Kohlenstoffatomen bedeutet,
$R_6$ Wasserstoff, Alkyl mit 1 bis 4 Kohlenstoffatomen, Alkoxy mit 1 bis 4 Kohlenstoffatomen, Halogen oder, falls $R_5$ Wasserstoff ist, auch Nitro oder Trifluormethyl bedeutet,
$R_7$ Wasserstoff oder Alkyl mit 1 bis 4 Kohlenstoffatomen bedeutet,
$R_8$ Wasserstoff oder Alkyl mit 1 bis 4 Kohlenstoffatomen bedeutet, oder
$R_7$ und $R_8$ zusammen mit dem Stickstoffatom, an das sie gebunden sind, eine heterocyclische Gruppe b bilden,

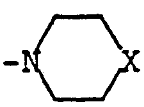

b

worin
X für eine Bindung, $-CH_2-$, $C_2H_4-$, O oder S steht,
Z eine Alkylenkette mit 2 bis 5 Kohlenstoffstomen bedeutet, welche gegebenenfalls an einem nicht an Stickstoff gebundenen Kohlenstoff durch Hydroxy oder eine $R_1O$-Gruppe, worin $R_1$ die oben angegebene Bedeutung besitzt, substituiert ist,
sowie deren Säureadditionssalze.

2. 1-Phenyl-2-aminocarbonylindol-Verbindungen gemäß Anspruch 1, worin Z und $R_1$ bis $R_6$ die in Anspruch 1 angegebene Bedeutung besitzen, $R_7$ Wasserstoff oder Alkyl mit 1 bis 4 Kohlenstoffatomen bedeutet und $R_8$ Wasserstoff oder Alkyl mit 1 bis 4 Kohlenstoffatomen bedeutet.

3. 3-Acyloxy-1-phenyl-2-aminocarbonylindol-Verbindungen gemäß Anspruch 1, worin Z und $R_1$ die in Anspruch 1 angegebene Bedeutung besitzen, $R_2$ Wasserstoff bedeutet, $R_3$ Wasserstoff oder Halogen bedeutet, $R_4$ Wasserstoff oder Halogen bedeutet, $R_5$ Wasserstoff, Halogen oder Alkyl mit 1 bis 4 Kohlenstoffatomen bedeutet, $R_6$ Wasserstoff, Halogen oder Alkyl mit 1 bis 4 Kohlenstoffatomen bedeutet, $R_7$ Wasserstoff oder Alkyl mit 1 bis 4 Kohlenstoffatomen bedeutet und $R_8$ Wasserstoff oder Alkyl mit 1 bis 4 Kohlenstoffatomen bedeutet.

4. 3-Acyloxy-1-phenyl-2-aminocarbonylindol-Verbindungen gemäß Anspruch 3, worin $R_1$ bis $R_8$ die in Anspruch 3 angegebene Bedeutung besitzen und Z eine Alkylenkette mit 2 bis 5 Kohlenstoffatomen, welche an einem nicht an Stickstoff gebundenen Kohlenstoff durch Hydroxy oder eine $R_1O$-Gruppe substituiert ist, bedeutet.

5. 3-Acyloxy-1-phenyl-2-aminocarbonylindol-Verbindungen gemäß Anspruch 4, worin $R_1$, $R_7$, $R_8$ und Z die in Anspruch 4 angegebene Bedeutung besitzen, und $R_2$ bis $R_6$ Wasserstoff bedeuten.

6. 3-Acyloxy-1-phenyl-2-aminocarbonylindol-Verbindungen gemäß Anspruch 1, worin $R_2$ bis $R_8$ und Z die in Anspruch 1 angegebene Bedeutung besitzen und $R_1$ eine Alkanoylgruppe mit 2 bis 5 Kohlenstoffatomen bedeutet, welche gegebenenfalls substituiert sein kann durch eine Carboxyl- oder Alkoxycarbonylgruppe, deren Alkoxyrest 1 bis 4 Kohlenstoffatome besitzt, oder Alkoxy mit 1 bis 4 Kohlenstoffatomen oder Benzoyloxy.

7. 3-Acyloxy-1-phenyl-2-aminocarbonylindol-Verbindungen gemäß Anspruch 6, worin $R_2$ bis $R_4$ und Z die in Anspruch 6 angegebene Bedeutung besitzen und $R_1$ eine Alkanoylgruppe mit 2 bis 5 Kohlenstoffatomen bedeutet.

8. 3-Acyloxy-1-phenyl-2-aminocarbonylindol-Verbindungen gemäß Anspruch 1, worin $R_2$ bis $R_8$ und Z die in Anspruch 1 angegebene Bedeutung besitzen und $R_1$ eine Gruppe a

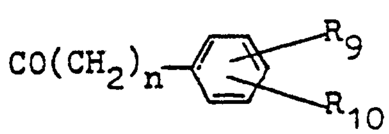

a

bedeutet, worin n 0 oder 1 ist und $R_9$ und $R_{10}$ unabhängig voneinander Wasserstoff, Halogen, Alkyl mit 1 bis 4 Kohlenstoffatomen oder Alkoxy mit 1 bis 4 Kohlenstoffatomen darstellen, oder, falls n 0 ist und $R_9$ Wasserstoff

darstellt, $R_{10}$ auch Nitro oder Trifluormethyl bedeutet.

9. 3-Acyloxy-1-phenyl-2-aminocarbonylindol-Verbindungen gemäß Anspruch 3, worin $R_2$ bis $R_8$ und Z die in Anspruch 3 angegebene Bedeutung besitzen und $R_1$ eine Alkanoylgruppe mit 2 bis 5 Kohlenstoffatomen bedeutet.

10. 3-Acyloxy-1-phenyl-2-aminocarbonylindol-Verbindungen gemäß Anspruch 3, worin $R_2$ bis $R_8$ und Z die in Anspruch 3 angegebene Bedeutung besitzen und $R_1$ eine Gruppe a

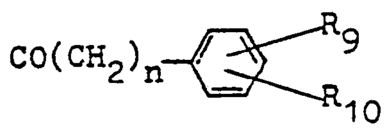 a

bedeutet, worin n 0 oder 1 ist und $R_9$ und $R_{10}$ unabhängig voneinander Wasserstoff, Halogen, Alkyl mit 1 bis 4 Kohlenstoffatomen oder Alkoxy mit 1 bis 4 Kohlenstoffatomen darstellen, oder, falls n 0 ist und $R_9$ Wasserstoff darstellt, $R_{10}$ auch Nitro oder Trifluormethyl bedeutet.

11. 3-Acyloxy-1-phenyl-2-aminocarbonylindol-Verbindungen gemäß Anspruch 1, worin $R_2$ bis $R_6$ Wasserstoff bedeuten, $R_7$ und $R_8$ Äthyl bedeuten, Z die 2-Hydroxypropylengruppe bedeutet und $R_1$ für Pivaloyl oder Benzoyl steht.

12. 3-Acyloxy-1-phenyl-2-aminocarbonylindol-Verbindungen gemäß Anspruch 1, worin $R_2$ bis $R_6$ Wasserstoff bedeuten, $R_7$ und $R_8$ Äthyl bedeuten, Z die 2-Hydroxypropylengruppe oder eine $R_1O$-Propylengruppe bedeutet und $R_1$ für Acetyl. Propionyl, Isobutyryl, Pivaloyl, Dodecanoyl, Benzoyl, Chlorbenzoyl, Nitrobenzoyl, Methylbenzoyl, Methoxybenzoyl oder Phenylacetyl steht.

**Claims**

1. 1-Phenyl-2-aminocarbonyl indole compounds of the general Formula I

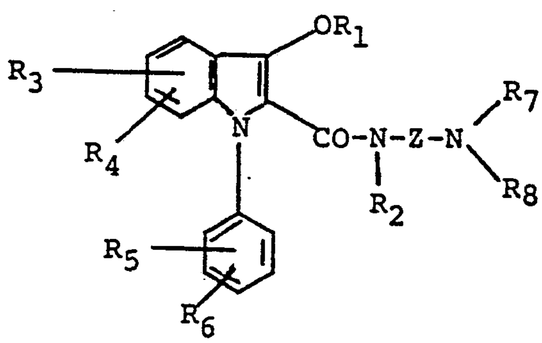 I

in which

$R_1$ is an alkanoyl group with 2 to 12 carbon atoms, which may be substituted if required by an alkoxy radical with 1 to 4 carbon atoms or a carboxyl or alkoxycarbonyl group, in which the alkoxycarbonyl group has an alkoxy radical with 1 to 4 carbon atoms, or

$R_1$ is a group a

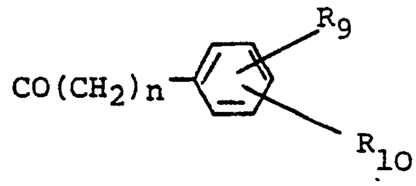

a

in which n is 0, 1, 2 or 3 and $R_9$ and $R_{10}$ independently of each other represent hydrogen, halogen, alkyl with 1 to 4 carbon atoms or alkoxy with 1 to 4 carbon atoms or, if they are linked to 2 adjacent carbon atoms, also together represent methylenedioxy, or, if n is 0 and $R_9$ represents hydrogen, $R_{10}$ also denotes nitro or trifluoromethyl,

$R_2$ is hydrogen or alkyl with 1 to 4 carbon atoms,

$R_3$ is hydrogen, halogen, alkyl with 1 to 4 carbon atoms or alkoxy with 1 to 4 carbon atoms,

$R_4$ is hydrogen, halogen, alkyl with 1 to 4 carbon atoms or alkoxy with 1 to 4 carbon atoms,

$R_5$ is hydrogen, alkyl with 1 to 4 carbon atoms, halogen or alkoxy with 1 to 4 carbon atoms,

$R_6$ is hydrogen, alkyl with 1 to 4 carbon atoms, alkoxy with 1 to 4 carbon atoms, halogen, or, if $R_5$ is hydrogen, is also nitro or trifluoromethyl,

$R_7$ is hydrogen or alkyl with 1 to 4 carbon atoms,

$R_8$ is hydrogen or alkyl with 1 to 4 carbon atoms, or

$R_7$ and $R_8$ together with the nitrogen atom, to which they are linked, form a heterocyclic group b

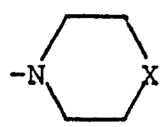

b

in which

X stands for a bond, $-CH_2-$, $C_2H_4-$, O or S,

Z is an alkylene chain with 2 to 5 carbon atoms which, if required, at a carbon not linked to nitrogen is substituted by hydroxy or an $R_1$ O-group, in which $R_1$ has the meaning given above,

and their acid addition salts.

2. 1-Phenyl-2-aminocarbonyl indole compounds according to Claim 1, in which Z and $R_1$ to $R_6$ have the meanings given in Claim 1, $R_7$ is hydrogen or alkyl with 1 to 4 carbon atoms and $R_8$ is hydrogen or alkyl with 1 to 4 carbon atoms.

3. 3-Acyloxy-1-phenyl-2-aminocarbonyl indole compounds according to Claim 1, in which Z and $R_1$ have the meanings given in Claim 1, $R_2$ is hydrogen, $R_3$ is hydrogen or halogen, $R_4$ is hydrogen or halogen, $R_5$ is hydrogen, halogen or alkyl with 1 to 4 carbon atoms, $R_6$ is hydrogen, halogen or alkyl with 1 to 4 carbon atoms, $R_7$ is hydrogen or alkyl with 1 to 4 carbon atoms and $R_8$ is hydrogen or alkyl with 1 to 4 carbon atoms.

4. 3-Acyloxy-1-phenyl-2-aminocarbonyl indole compounds according to Claim 3, in which $R_1$ to $R_8$ have the meanings given in Claim 3, and Z is an alkylene chain with 2 to 5 carbon atoms, which at a carbon not linked to nitrogen is substituted by hydroxy or a $R_1O$-group.

5. 3-Acyloxy-1-phenyl-2-aminocarbonyl indole compounds according to Claim 4, in which $R_1$, $R_7$, $R_8$ and Z have the meanings given in Claim 4, and $R_2$ to $R_6$ denote hydrogen.

6. 3-Acyloxy-1-phenyl-2-aminocarbonyl indole compounds according to Claim 1, in which $R_2$ to $R_8$ and Z have the meanings given in Claim 1 and $R_1$ is an alkanoyl group with 2 to 5 carbon atoms, which may be substituted if required by a carboxyl or alkoxycarbonyl group the alkoxy radical of which has 1 to 4 carbon atoms, or alkoxy with 1 to 4 carbon atoms or benzoyloxy.

7. 3-Acyloxy-1-phenyl-2-aminocarbonyl indole compounds according to Claim 6, in which $R_2$ to $R_4$ and Z have the meaning given in Claim 6, and $R_1$ denotes an alkanoyl group with 2 to 5 carbon atoms.

8. 3-Acyloxy-1-phenyl-2-aminocarbonyl indole compounds , according to Claim 1, in which $R_2$ to $R_8$ and Z have the meanings given in Claim 1 and $R_1$ is a group a

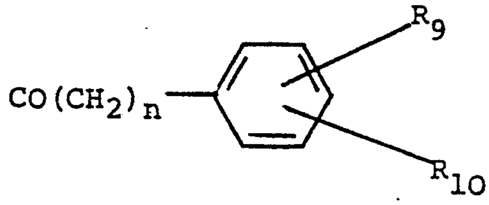

a

in which n is 0 or 1 and $R_9$ and $R_{10}$ independently of each other represent hydrogen, halogen, alkyl with 1 to 4 carbon atoms or alkoxy with 1 to 4 carbon atoms, or, if n is 0 and $R_9$ represents hydrogen, $R_{10}$ is also nitro or trifluoromethyl.

9. 3-Acyloxy-1-phenyl-2-aminocarbonyl indole compounds according to Claim 3, in which $R_2$ and $R_8$ and Z have the meanings given in Claim 3, and $R_1$ is an alkanoyl group with 2 to 5 carbon atoms.

10. 3-Acyloxy-1-phenyl-2-aminocarbonyl indole compounds according to Claim 3, in which $R_2$ to $R_8$ and Z have the meanings given in Claim 3 and $R_1$ is a group a

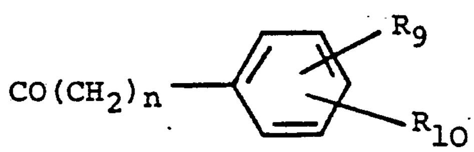

a

in which n is 0 or 1 and $R_9$ and $R_{10}$ independently of each other represent hydrogen, halogen, alkyl with 1 to 4 carbon atoms or alkoxy with 1 to 4 carbon atoms, or, if n is 0 and $R_9$ represents hydrogen, $R_{10}$ is also nitro or trifluoromethyl.

11. 3-Acyloxy-1-phenyl-2-aminocarbonyl indole compounds according to Claim 1, in which $R_2$ to $R_6$ are hydrogen, $R_7$ and $R_8$ are ethyl, Z is the 2-hydroxypropylene group and $R_1$ stands for pivaloyl or benzoyl.

12. 3-Acyloxy-1-phenyl-2-aminocarbonyl indole compounds according to Claim 1, in which $R_2$ to $R_6$ are hydrogen, $R_7$ and $R_8$ are ethyl, Z is the 2-hydroxypropylene group or an $R_1O$-propylene group and $R_1$ stands for acetyl, propionyl, isobutyryl, pivaloyl, dodecanoyl, benzoyl, chlorobenzoyl, nitrobenzoyl, methylbenzoyl, methoxybenzoyl or phenylacetyl.

**Revendications**

1. 1-phényl-2-aminocarbonylindoles de formule générale I:

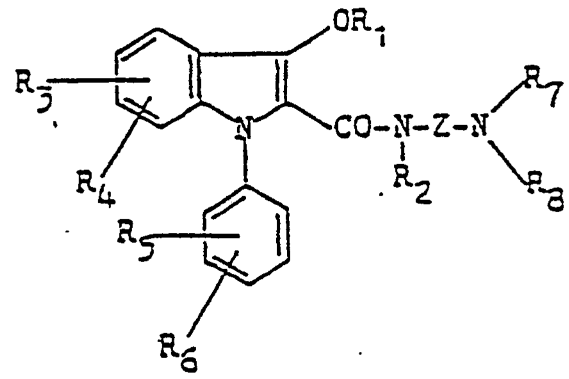

I

dans laquelle

$R_1$ représente un groupe alcanoyle ayant 2 à 12 atomes de carbone, qui peut éventuellement être substitué par un reste alcoxy ayant 1 à 4 atomes de carbone ou par un groupe carboxyle ou alcoxycarbonyle, ce groupe alcoxycarbonyle possédant un reste elcoxy ayant 1 à 4 atomes de carbone, ou bien

$R_1$ représente un groupe a

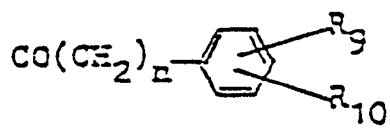

a

dans laquelle n vaut 0, 1, 2 ou 3 et $R_9$ et $R_{10}$ représentent chacun, indépendamment l'un de l'autre, un atome d'hydrogène ou d'halogène, un groupe alkyle ayant 1 à 4 atomes de carbone ou alcoxy ayant 1 à 4 atomes de carbone ou, s'ils sont fixés sur 2 atomes de carbone voisins, ils peuvent représenter ensemble aussi un groupe méthylènedioxy ou, si n est nul et si $R_9$ représente un atome d'hydrogène, $R_{10}$ représente également un groupe nitro ou trifluorométhyle,

$R_2$ représente un atome d'hydrogène ou un groupe alkyle ayant 1 à 4 atomes de carbone,

$R_3$ représente un atome d'hydrogène ou d'halogène ou un groupe alkyle ayant 1 à 4 atomes de carbone ou alcoxy ayant 1 à 4 atomes de carbone,

$R_4$ représense un atome d'hydrogène ou d'halogène, un groupe alkyle ayant 1 à 4 atomes de carbone ou alcoxy ayant 1 à 4 atomes de carbone,

$R_5$ représente un atome d'hydrogène, un groupe alkyle ayant 1 à 4 atomes de carbone, un atome d'halogène ou un groupe alcoxy ayant 1 à 4 atomes de carbone,

$R_6$ représente un atome d'hydrogène, un groupe alkyle ayant 1 à 4 atomes de carbone, un groupe alcoxy ayant 1 à 4 atomes de carbone, un atome d'halogène ou bien, si $R_5$ représente un atome d'hydrogène, $R_6$ peut représenter également un groupe nitro ou trifluorométhyle,

$R_7$ représente un atome d'hydrogène ou un groupe alkyle ayant 1 à 4 atomes de carbone,

$R_8$ représente un atome d'hydrogène ou un groupe alkyle ayant 1 à 4 atomes de carbone, ou

$R_7$ et $R_8$ pris avec l'atome d'azote auquel ils sont fixés, forment un groupe hétérocyclique b

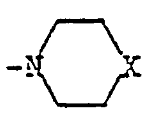

b

dans lequel

X représente une liaison, $-CH_2-$, $C_2H_4-$, O ou S,

Z représente une chaîne alkylène ayant 2 à 5 atomes de carbone, qui peut éventuellement être substituée, sur un atome de carbone non relié à l'azote, par un groupe hydroxy ou par un groupe $R_1O$, dans lequel $R_1$ a le

sens indiqué ci-dessus,

ainsi que leurs sels d'addition d'acide.

2. 1-phényl-2-aminocarbonylindoles selon la revendication 1, dans lesquels Z et $R_1$ à $R_6$ ont le sens indiqué à la revendication 1, $R_7$ représente un atome d'hydrogène ou un groupe alkyle ayant 1 à 4 atomes de carbone, et $R_8$ représente un atome d'hydrogène ou un groupe alkyle ayant 1 à 4 atomes de carbone.

3. 3-acyloxy-1-phényl-2-aminocarbonylindoles selon la revendication 1, dans lesquels Z et $R_1$ ont le sens indiqué à la revendication 1, $R_2$ représente un atome d'hydrogène, $R_3$ représente un atome d'hydrogène ou d'halogène, $R_4$ représente un atome d'hydrogène ou d'halogène, $R_5$ représente un atome d'hydrogène ou d'halogène ou un groupe alkyle ayant 1 à 4 atomes de carbone, $R_6$ représente un atome d'hydrogène ou d'halogène ou un groupe alkyle ayant 1 à 4 atomes de carbone, $R_7$ représente un atome d'hydrogène ou un groupe alkyle ayant 1 à 4 atomes de carbone et $R_8$ représente un atome d'hydrogène ou un groupe alkyle ayant 1 à 4 atomes de carbone.

4. 3-acyloxy-1-phényl-2-aminocarbonylindoles selon la revendication 3, dans lesquels $R_1$ à $R_8$ ont le sens indiqué à la revendication 3, et Z représente une chaîne alkylène ayant 2 à 5 atomes de carbone, qui est substituée, sur un atome de carbone non lié à l'azote, par un groupe hydroxy ou par un groupe $R_1O$.

5. 3-acyloxy-1-phényl-2-aminocarbonylindoles selon la revendication 4, dans lesquels $R_1$, $R_7$, $R_8$ et Z ont le sens indiqué à la revendication 4, et $R_2$ à $R_6$ représentent chacun un atome d'hydrogène.

6. 3-acyloxy-1-phényl-2-aminocarbonylindoles selon la revendication 1, dans lesquels $R_2$ à $R_8$ ainsi que Z ont le sens indiqué à la revendication 1, et $R_1$ représente un groupe alcanoyle ayant 2 à 5 atomes de carbone, qui peut éventuellement être substitué par un groupe carboxyle ou alcoxycarbonyle dont le reste alcoxy comporte 1 à 4 atomes de carbone, ou par un groupe alcoxy ayant 1 à 4 atomes de carbone ou benzoyloxy.

7. 3-acyloxy-1-phényl-2-aminocarbonylindoles selon la revendication 6, dans lesquels $R_2$ à $R_4$ ainsi que Z ont le sens indiqué à la revendication 6, et $R_1$ représente un groupe alcanoyle ayant 2 à 5 atomes de carbone.

8. 3-acyloxy-1-phényl-2-aminocarbonylindoles selon la revendication 1, dans lesquels $R_2$ à $R_8$ ainsi que Z ont le sens indiqué à la revendication 1, et $R_1$ représente un groupe a

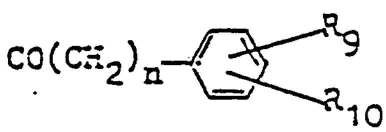 a

dans laquelle n vaut 0 ou 1 et $R_9$ et $R_{10}$ représentent chacun, indépendamment l'un de l'autre, un atome d'hydrogène ou d'halogène, un groupe alkyle ayant 1 à 4 atomes de carbone ou alcoxy ayant 1 à 4 atomes de carbone ou bien, si n est nul et si $R_9$ représente un atome d'hydrogène, $R_{10}$ peut aussi représenter un groupe nitro ou trifluorométhyle.

9. 3-acyloxy-1-phényl-2-aminocarbonylindoles selon la revendication 3, dans lesquels $R_2$ à $R_8$ ainsi que Z ont le sens indiqué à la revendication 3, et $R_1$ représente un groupe alcanoyle ayant 2 à 5 atomes de carbone.

10. 3-acyloxy-1-phényl-2-aminocarbonylindoles selon la revendication 3, dans lesquels $R_2$ à $R_8$ et Z ont le sens indiqué à la revendication 3, et $R_1$ représente un groupe a

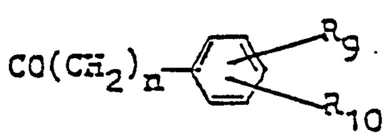 a

dans laquelle n vaut 0 ou 1 et $R_9$ et $R_{10}$ représentent chacun, indépendamment l'un de l'autre, un atome d'hydrogène ou d'halogène, un groupe alkyle ayant 1 à 4 atomes de carbone ou alcoxy ayant 1 à 4 atomes de carbone ou bien, si n est nul et si $R_9$ représente un atome d'hydrogène, $R_{10}$ peut représenter aussi un groupe nitro ou trifluorométhyle.

11. 3-acyloxy-1-phényl-2-aminocarbonylindoles selon la revendication 1, dans lesquels $R_2$ à $R_6$ représentent chacun un atome d'hydrogène, $R_7$ et $R_8$ représentent chacun un groupe éthyle, Z représente un groupe 2-hydroxypropylène et $R_1$ représente un groupe pivaloyle ou benzoyle.

12. 3-acyloxy-1-phényl-2-aminocarbonylindoles selon la revendication 1, dans lesquels $R_2$ à $R_6$ représentent chacun un atome d'hydrogène, $R_7$ et $R_8$ représentent chacun un groupe éthyle, Z représente le groupe 2-hydroxypropylène ou un groupe $R_1O$-propylène, et $R_1$ représente un groupe acétyle, propionyle, isobutyryle, pivaloyle, dodécanoyle, benzoyle, chlorobenzoyle, nitrobenzoyle, méthylbenzoyle, méthoxybenzoyle ou phénylacétyle.